# EUROPEAN PATENT APPLICATION

(11) **EP 1 493 428 A1**
(43) Date of publication of application: **05.01.2005**
(21) Application number: 04253944.5
(22) Date of filing: 30.06.2004
(51) Int. Cl.: A61K 7/155, A61K 7/48

(54) **Method for removing hair**

(30) Priority: 01.07.2003 US 611100
(71) Applicant: Johnson & Johnson Consumer Companies, Inc., Skillman, New Jersey 08858 (US)
(72) Inventor: Halas, Lynn, Ewing, New Jersey 08638 (US); Lu, Jue-Chen, Belle Mead, New Jersey 08502 (US); Skover, Gregory, Princeton, New Jersey 08540 (US)
(74) Representative: Fisher, Adrian John

(57) **Abstract**

The invention features a method for removing hair by: topically applying to an area of skin containing the hair a safe and effective amount of a composition containing at least one legume product; and exposing the area of skin to an effective amount of light to remove said hair.

## Description

### FIELD OF THE INVENTION

The present invention relates to a method for removing hair. The method includes the topical application of compositions prepared from legumes prior to laser hair removal treatment.

### BACKGROUND OF THE INVENTION

Consumers have utilized depilatory agents, such as thioglycolic acid to remove unwanted hair for many years. The depilatory agents function by chemically destroying the hair above the skin. Although depilatory agents are effective at hair removal, they tend to have offensive odors and their effect is temporary, as the hair grows back over time.

The use of light, whether coherent or non-coherent, for hair removal has been recently introduced. Laser has some benefits over depilatory agents. One benefit of the use of such light, such as laser light, is that it is more effective at removing hair. Another benefit is that laser treatment does not result in the generation of an offensive odor.

Although laser is quite effective at removing hair, there are still some problems associated with the use of laser hair removal. For example, laser treatment can be quite expensive, and the treatment can also be painful. Therefore, there is a need to improve laser hair removal in order to enhance its performance.

### SUMMARY OF THE INVENTION

In one aspect, the invention features a method for removing hair by: topically applying to an area of skin containing the hair a safe and effective amount of a composition containing at least one legume product; and exposing the area of skin to an effective amount of light to remove said hair.

In another aspect, the invention features a method for minimizing the rate of hair growth or the amount of hair growth following light treatment by: topically applying to an area of skin containing the hair a safe and effective amount of a composition containing at least one legume product; and exposing the area of skin to an effective amount of light to remove said hair.

In another aspect, the invention features a method for reducing the frequency of hair removal by light treatment by: topically applying to an area of skin containing the hair a safe and effective amount of a composition containing at least one legume product; and exposing the area of skin to an effective amount of light to remove said hair.

In another aspect, the invention features a method for minimizing visibility of hair following light treatment by: topically applying to an area of skin containing the hair a safe and effective amount of a composition containing at least one legume product; and exposing the area of skin to an effective amount of light to remove said hair.

In another aspect, the invention features a method of promoting a composition containing a legume product by instructing the user of the composition to topically apply the composition to an area of skin containing hair prior to or after exposure of the area of skin to an effective amount of light to remove said hair.

### DETAILED DESCRIPTION OF THE INVENTION

It is believed that one skilled in the art can, based upon the description herein, utilize the present invention to its fullest extent. The following specific embodiments are to be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs. Also, all publications, patent applications, patents, and other references mentioned herein are incorporated by reference. Whenever used, any percentage is weight by weight (w/w) unless otherwise indicated.

What is meant by a "packaged product" is a product containing a composition in finished packaged form. In one embodiment, the package is a container such as a plastic, metal or glass tube or jar containing the composition. The container may further include an applicator to topically apply the composition to the skin or hair such as a wipe. The packaged product may further contain additional packaging such as a plastic or cardboard box for storing such container. In one embodiment, the packaged product contains instructions directing the purchaser and/or user of the composition to topically apply the composition to an area of skin (e.g., areas of skin containing unwanted hair such as the face, legs, arms, back, or pubic region) containing hair prior to or after exposure of the area of skin to laser light.

What is meant by "promoting" is promoting, advertising, or marketing. Examples of promoting include, but are not limited to, written, visual, or-verbal statements made on or in the packaging containing the composition the product or in stores, magazines, newspaper, radio, television, internet, and the like. Examples of such statements include, but are not limited to: apply prior to light treatment, apply immediately after light treatment, apply between light treatment, delay hair growth, delay hair re-growth, reduce the appearance of hair, decrease hair coarseness, reduce hair thickness, reduce frequency of light treatments, and minimize side effects of light treatment.

As used herein, "topically applying" means directly laying on or spreading on outer skin or hair, e.g., by use of the hands or an applicator such as a wipe, puff, roller, or spray.

As used herein, "cosmetically-acceptable" means that the product(s), compound(s), or composition(s) which the term describes are suitable for use in contact with tissues (e.g., the skin or hair) without undue toxicity, incompatibility, instability, irritation, allergic response, and the like. This term is not intended to limit the compound/product/composition to which it describes for use solely as a cosmetic (e.g., the ingredient/product may be used as a pharmaceutical).

As used herein, "topical carrier" means one or more compatible solid or liquid filler diluents that are suitable for topical administration to a mammal. Examples of topical carriers include, but are not limited to, water, waxes, oils, emollients, emulsifiers, thickening agents, gelling agents, and mixtures thereof.

As used herein, "safe and effective amount" means an amount of product(s), compound(s), or composition(s) (e.g., the legume product or composition containing the legume product) sufficient to induce a positive modification in laser hair removal, but low enough to avoid serious side effects. The safe and effective amount of the product(s), compound(s), or composition(s) will vary with the amount of hair present, the age and physical condition of the end user, the duration of the treatment, the specific compound, product, or composition employed, the particular cosmetically-acceptable carrier utilized, and like factors.

### Legume Product

What is meant by a "legume product" is a substance derived from a legume fruit. A legume is a plant from the family Leguminosae, which has a dehiscent fruit such as a bean, pea, or lentil. Examples of legumes, include but are not limited to, beans such as soybeans, lentil beans, peas, and peanuts.

The legume product may contain the entire legume fruit (e.g., the legume fruit ground into a powder) or only a portion of the legume (e.g., an extract of the legume).

The legume product may be in the form of a fluid (e.g., legume fruit milk, which is a mixture of the legume fruit powder and water) or a solid (e.g., legume fruits powders or legume fruit milk powder such as soy milk powder). When in the form of a fluid, the term "legume product" refers to the solid constituents of the fluid derived from the legume.

The compositions utilized in the present invention comprise a safe and effective amount of the legume product (e.g., soy product). The compositions may contain from about 0.0 1 % to about 50%, such as from about 0.1 % to about 20% or from about 1% to about 10%, by weight, of the legume product (e.g., a soy product).

### Soy Product

What is meant by a "Soy Product" is a substance derived from the soybean. The soy product may contain only a portion of the soybean (e.g., an extract of the soybean such as a lipid reduced soybean powder or filtered soymilk) or may contain the entire soybean (e.g., a ground powder of the legume). The soy product may be in the form of a fluid (e.g., soymilk) or a solid (e.g., a soybean powder or soymilk powder). When in the form of a fluid, the term "soy product" refers to the solid constituents of the fluid that are derived from the soybean.

The soy product may be soybean powder. Soybean powder may be made by grinding dry soybeans. The soybean powder may be lyophilized.

Soymilk and soymilk powder are also useful soy products. Soymilk is a combination of solids derived from soybeans and water, the mixture of which has some or all of the insoluble constituents filtered off. Soymilk powder is evaporated soymilk, which in one embodiment, is in a lyophilized or spray-dried form. Procedures for manufacturing soymilk include, but are not limited to, the following three procedures. First, soymilk may be made by placing soybeans into water to allow them to absorb the water. The swelled beans are then ground and additional water is then added. The mixture may then be filtered to remove any insoluble residue. Second, soymilk may also be prepared from soybean powder. Soybean powder is thoroughly mixed with water (e.g., for at least one hour), which may then be followed by a filtration process to remove insoluble residues. Third, soymilk can also be reconstituted from soymilk powder by adding water. The soymilk may comprise from between about 1% to about 50%, by weight (e.g., from about 5% to about 20%, by weight) of solids from the soybean.

### Anti-microbial Treatment of Legume Product

The surface of legume fruits often contain high levels of microorganisms. Thus, prior to use by humans, the legume product needs to be treated to reduce or eliminate such microorganisms.

The legume products utilized in the present invention may have a total microbial content of less than about 10,000 colony-forming units ("cfu") per gram. Preferably, the soy products utilized in the present invention have a microbial content of less than about 1,000 cfu per gram (such as less than about 100 cfu per gram) of the legume product.

The legume products utilized in the present invention may have a total objectionable microbial content of less than 300 cfu per gram such as less than 150 cfu per gram. Preferably, the legume products utilized in the present invention have an undetectable amount of any objectionable microbials for at least one gram (e.g., at least ten grams) of legume product.

The legume product may be exposed to gamma irradiation. The legume product may be exposed to between about 2 to about 30 kGy of gamma irradiation, such as between about 5 and about 10 kGy of gamma irradiation. Such treatment reduces the microbial content of the legume product, while maintaining its biological activity (e.g., serine protease inhibitory activity). The treatment of legume products with gamma irradiation maintains the cosmetic elegance of the legume product, such as maintained natural colors and does not induce significant malodors.

Other anti-microbial processes that also maintain the protease inhibitory activity of the legume product that can be practiced alone or in combination with gamma irradiation, include, but are not limited to, exposure to x-rays, high energy electron or proton beams, ultraviolet radiation, hydrostatic pressure, and addition of chemical agents possessing antimicrobial activity, and combinations thereof.

### Topical Compositions

The topical compositions useful in the present invention involve formulations suitable for topical application to skin. The composition may comprise the soy product and a cosmetically-acceptable topical carrier. The cosmetically-acceptable topical carrier may comprise from about 50% to about 99.99%, by weight, of the composition (e.g., from about 80% to about 95%, by weight, of the composition).

The compositions may be made into a wide variety of product types that include but are not limited to solid and liquid compositions such as lotions, creams, gels, sticks, sprays, shaving creams, ointments, cleansing liquid washes and solid bars, shampoos, pastes, powders, mousses, shaving creams, adhesive strips, and wipes. These product types may comprise several types of cosmetically acceptable topical carriers including, but not limited to solutions, emulsions (e.g., microemulsions and nanoemulsions), gels, solids and liposomes. The following are non-limitative examples of such carriers. Other carriers can be formulated by those of ordinary skill in the art.

The topical compositions useful in the present invention can be formulated as solutions. Solutions typically include an aqueous solvent (e.g., from about 50% to about 99.99% or from about 90% to about 99% of a cosmetically acceptable aqueous solvent).

Topical compositions useful in the subject invention may be formulated as a solution comprising an emollient. Such compositions preferably contain from about 2% to about 50% of an emollient(s). As used herein, "emollients" refer to materials used for the prevention or relief of dryness, as well as for the protection of the skin. A wide variety of suitable emollients are known and may be used herein. See International Cosmetic Ingredient Dictionary and Handbook, eds. Wenninger and McEwen, pp. 1656-61, 1626, and 1654-55 (The Cosmetic, Toiletry, and Fragrance Assoc., Washington, D.C., 7^{th} Edition, 1997) (hereinafter "INCI Handbook") contains numerous examples of suitable materials.

A lotion can be made from such a solution. Lotions typically comprise from about 1% to about 20% (e.g., from about 5% to about 10%) of an emollient(s) and from about 50% to about 90% (e.g., from about 60% to about 80%) of water.

Another type of product that may be formulated from a solution is a cream. A cream typically comprises from about 5% to about 50% (e.g., from about 10% to about 20%) of an emollient(s) and from about 45% to about 85% (e.g., from about 50% to about 75%) of water.

Yet another type of product that may be formulated from a solution is an ointment. An ointment may comprise a simple base of animal or vegetable oils or semi-solid hydrocarbons. An ointment may comprise from about 2% to about 10% of an emollient(s) plus from about 0.1% to about 2% of a thickening agent(s). A more complete disclosure of thickening agents or viscosity increasing agents useful herein can be found in the INCI Handbook pp. 1693-1697.

The topical compositions useful in the present invention may be formulated as emulsions. If the carrier is an emulsion, from about 1% to about 10% (e.g., from about 2% to about 5%) of the carrier comprises an emulsifier(s). Emulsifiers may be nonionic, anionic or cationic. Suitable emulsifiers are disclosed in, for example, INCI Handbook, pp.1673-1686.

Lotions and creams can be formulated as emulsions. Typically such lotions comprise from 0.5% to about 5% of an emulsifier(s). Such creams would typically comprise from about 1% to about 20% (e.g., from about 5% to about 10%) of an emollient(s); from about 20% to about 80% (e.g., from 30% to about 70%) of water; and from about 1% to about 10% (e.g., from about 2% to about 5%) of an emulsifier(s).

Single emulsion skin care preparations, such as lotions and creams, of the oil-in-water type and water-in-oil type are well-known in the cosmetic art and are useful in the subject invention. Multiphase emulsion compositions, such as the water-in-oil-in-water type are also useful in the subject invention. In general, such single or multiphase emulsions contain water, emollients, and emulsifiers as essential ingredients.

The topical compositions of this invention can also be formulated as a gel (e.g., an aqueous gel using a suitable gelling agent(s)). Suitable gelling agents for aqueous gels include, but are not limited to, natural gums, acrylic acid and acrylate polymers and copolymers, and cellulose derivatives (e.g., hydroxymethyl cellulose and hydroxypropyl cellulose). Suitable gelling agents for oils (such as mineral oil) include, but are not limited to, hydrogenated butylene/ethylene/styrene copolymer and hydrogenated ethylene/propylene/styrene copolymer. Such gels typically comprise between about 0.1% and 5%, by weight, of such gelling agents.

The topical compositions of the present invention can also be formulated into a solid formulation (e.g., a wax-based stick, soap bar composition, powder, or a wipe containing powder).

The topical compositions useful in the subject invention may contain, in addition to the aforementioned components, a wide variety of additional oil-soluble materials and/or water-soluble materials conventionally used in compositions for use on skin, hair, and nails at their art-established levels.

The topical compositions are applied to the area of skin containing such unwanted hair at a safe and effective level as discussed above. The topical compositions may be applied one or more times a day, preferably twice a day. The compositions may be applied prior to and/or after exposing the area of skin to laser light. In one embodiment, the composition is applied to the area of skin at least one day prior to exposing such area to laser light. When applied prior to laser treatment, the composition may be applied one day to about one month, such as from about one week to about two weeks, prior to laser treatment. The topical compositions may also be applied after laser treatment, e.g., on a daily basis, until the next laser treatment.

### Light Treatment

Any light (e.g., generated from a device) capable of hair removal is useful in the present invention. The light may be coherent or non-coherent. In one embodiment, light of sufficient intensity and/or duration is used to kill either the hair follicle and/or the skin tissue feeding the hair. In one embodiment, the light is laser light. Suitable lasers include, but are not limited to, alexandrite lasers such as Candela GentleLASE™ and the Cynosure Apogee™ lasers, diode lasers such as the Asclepion-Meditec MeDioStar™ lasers, ruby lasers such as the Asclepion-Meditec Ruby™ laser, and Nd:YAG Lasers such as the Continuum Medlite IV™ laser. The use of laser light to remove hair is well known in the art, see, e.g., U.S. Patent Nos. 6,544,255, 6,485,484, 6,273,885, 6,272,883, 6,217,572, and 6,045,548. Examples of non-laser light emitting devices include, but are not limited to, flash lamps and intense pulse light devices. Examples of such devices are disclosed in U.S. Pat. Nos. 5,683,380, 5,720,772, and 6,461,348.

In one embodiment, a photosensitizer is applied to the skin prior to exposure to laser light. Examples of photosensitizers include, but are not limited to, thermally acting and chemically acting photosensitizers. In one embodiment, the photosensitizer is illuminated with light at sufficient intensity and duration to photoactivate the photosensitizer, thereby producing heat and/or a chemical species, such as singlet oxygen, that damages the follicle and/or skin tissue feeding the hair so as to cause long term inhibition of hair growth from the follicle. Examples of such chemically acting photosensitizers include, but are not limited to, hematoporphyrin derivatives, zincphthalocyanine, butoxy silicon naphthalocyanine, or tin ethyl etiopurpurin and examples of thermally acting photosensitizers include, but are not limited to, methylene blue and indocyanine green. The use of photosensitizers is well known in the art, see, e.g., U.S. Patent Nos. 5,871,480 and 6,358,242.

### Example

A study was performed to determine whether there was an increased benefit in using a topically applied water-in-oil emulsion cream composition containing legume product, as described in Table 1, (the "Cream") in combination with laser treatment for the removal of unwanted hair. This study was an investigator blinded, randomized, split bikini region, controlled use trial. The study duration was twelve weeks, with the laser treatment at the end of the fourth week and at the end of the twelfth week. Eleven subjects were enrolled in the study. The subjects were women aged 21 - 53 yrs old, with Fitzpatrick skin types III-VI.

**Table 1**

| Brand Name | Chemical Name | % wt/wt |
|---|---|---|
| Dow Coming 5225C | Cyclomethicone/Dimethicone Copolyol | 10% |
| ABIL EM-97 | Dimethicone Copolyol (and) Cylcopentasiloxane | 1.5% |
| Dow Coming 9506 Powder | Dimethicone/Vinyldimethicone Cross polymer | 2.5% |
| BHT | butylated hydroxytoluene | 0.05% |
| Dow Coming 345 Fluid | Cyclomethicone | 18.5% |
| DI Water | Deionized water | 42.9% |
| Soymilk powder | Soymilk powder | 2% |
| Lactoferrin 100 | Lactoferrin | 1% |
| Dow Coming 2501 | Dimethicone Copolyol | 2% |
| Tween 20 | Polysorbate 20 | 1% |
| Sodium Chloride | sodium chloride | 0.5% |
| Benzyl Alcohol | Benzyl alcohol | 0.5% |
| Disodium EDTA | Disodium EDTA | 0.05% |
| Flavosterone SB | Isoflavones | 5% |
| Phenonip | Phenoxyethanol/Parabens | 1% |
| Dow Coming 345 Fluid | Cyclomethicone | 2.5% |
| Dow Coming 9040 Elastomer | Dimethicone/Vinyldimethicone Cross polymer | 9% |

The pubic region was the selected treatment region. The test area was defined as beginning along the inguinal fold and extending 10 cm along the fold itself and extending 5 cm onto the thigh. An adhesive template was attached to the skin of each subject to reproducibly define the test area bilaterally. Each subject had her left and right side bikini regions randomized for treatment. One side was treated twice a day throughout the study with the Cream while the other side was not so treated. Subjects were required to topically apply approximately 0.75 - 1 gram of the cream twice a day (AM and PM application) only on the specified side. Enrolled subjects were required to shave 4 days prior to each of their return visits. Subjects were also prohibited from using chemical depilatories or waxes or any other type of hair removal other than shaving. Subjects were asked to maintain their typical grooming behavior for the duration of the study.

The laser used was a long wave Q-switched Nd/YAG Laser (1064 nm, Candela Corporation, Wayland, MA) at a fluence of 16 J/cm2. Consumer self-assessment questionnaires were filled out during the 4, 8, and 12 week visits. In determining hair re-growth, hair counts (hairs measuring at least 2 mm) and hair specks (hairs measuring less than 2 mm) were counted. In determining percent of hair immediately after laser treatment, as compared to immediately before laser treatment, only hair counts (not hair specks) were included in the analysis. The reason for not including hair specks was because such specks generally fall out within a couple days after laser treatment.

The percent of hairs remaining over time (compared to baseline) was calculated and is reported in Table 2.

**Table 2**

| | 4 Week pre-treatment prior to Laser | Immediately after Laser treatment | 4 Weeks Post Laser | 8 Weeks Post Laser |
|---|---|---|---|---|
| Cream Applied | 73% | 19% | 30% | 41% |
| Cream Not Applied | 79% | 21% | 35% | 61% |
| Statistical p-value | Not significant | Not significant | 0.13 | 0.02 |

The topical application of a legume product, in combination with laser hair removal, was surprisingly found to effectively reduce hair count over time as compared to use of laser treatment alone. This result indicates that a topically applied legume product may enable consumers to require less frequent laser hair removal treatments.

The amount of hair re-growth that occurred after 1 laser treatment was also examined, and is summarized in Table 3.

**Table 3**

| | % Re-growth of Hair 8 weeks vs. 4 weeks Post Laser Treatment |
|---|---|
| Cream Applied | 34% |
| Cream Not Applied | 93% |

The combination of laser treatment with topically applied legume product yielded statistically less hair re-growth over time as compared to just laser treatment alone (p=0.04).

Another unexpected finding related to the amount of hair that was obliterated immediately after laser treatment. Pictures compared the amount of hair immediately before laser treatment to the amount of hair immediately after laser treatment. Hair was counted from the pictures taken and is summarized in Table 4.

**Table 4**

| | % Hair Remaining After Laser |
|---|---|
| Cream Applied | 17% |
| Cream Not Applied | 23% |

The side which received topical application of the legume product appeared to have less hair remaining immediately after laser treatment than the side treated with laser alone (p=0.1)

Subjects were also given self-assessment questionnaires to fill out during their 4, 8, and 12 week visits. Over time, subjects gave increasingly positive feedback. Subjects were asked to evaluate the treated side, versus the untreated side, on a scale (Strongly Disagree, Somewhat Disagree, Neither Agree Nor Disagree, Somewhat Agree, Strongly Agree). Responses in the top 2 categories (Somewhat Agree, Strongly Agree) were recorded. During the subjects' 4 week post laser treatment visit, 8/11 subjects rated the following attributes in the top 2 categories: made hair less noticeable/ less visible, made hair grow back slower, made less hair grow back. There were 9/11 subjects that felt that the soy treated side made the hair grow back slower, made less hair grow back, and made shaving easier.

It is understood that while the invention has been described in conjunction with the detailed description thereof, that the foregoing description is intended to illustrate and not limit the scope of the invention, which is defined by the scope of the appended claims. Other aspects, advantages, and modifications are within the claims.

## Claims

1. A method for removing hair comprising:
topically applying to an area of skin comprising said hair a safe and effective amount of a composition comprising at least one legume product; and
exposing said area of skin to an effective amount of light to remove said hair.

2. The method according to claim 1 wherein said legume product is a soy product.

3. The method of according to claim 1 or claim 2 wherein said composition is applied prior to exposing said area of skin to said light.

4. The method according to claim 3 wherein said composition is applied to the area of skin at least one day prior to exposing said area of skin to said light.

5. The method of according to claim 1 or claim 2 wherein said composition is applied after exposing said area of skin to said light.

6. The method according to any one of claims 1 to 5 wherein said composition is applied to said area of skin at least twice a day.

7. A method of promoting a composition comprising a legume product, said method comprising instructing the user of said composition to topically apply said composition to an area of skin comprising hair prior to or after exposure of said area of skin to an effective amount of light to remove said hair.

8. The method according to claim 7 wherein said legume product is a soy product.

9. The method of according to claim 7 or claim 8 wherein said method comprises instructing said user to topically apply said composition to said area of skin prior to exposure of said area of skin to said light.

10. The method of according to claim 7 or claim 8 wherein said method comprises instructing said user to topically apply said composition to said area of skin after exposure of said area of skin to said light.
